(19) Europäisches Patentamt / European Patent Office / Office européen des brevets

(11) **EP 4 599 837 A1**

(12) **EUROPEAN PATENT APPLICATION**

(43) Date of publication:
**13.08.2025 Bulletin 2025/33**

(21) Application number: **25157215.2**

(22) Date of filing: **11.02.2025**

(51) International Patent Classification (IPC):
**A61K 35/74** (2015.01)    **A23L 33/135** (2016.01)
**A61K 35/744** (2015.01)    **A61K 35/747** (2015.01)
**C12N 1/20** (2006.01)

(52) Cooperative Patent Classification (CPC):
**A61K 35/744; A23L 33/135; A61K 35/74;
A61K 35/747; C12N 1/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.02.2024 IT 202400002860**

(71) Applicant: **Proge Farm S.r.l.
28100 Novara (NO) (IT)**

(72) Inventors:
- **DONDI, Giancarla
  28100 Novara (IT)**
- **USORINI, Paola
  28100 Novara (IT)**

(74) Representative: **Trupiano, Federica
Marietti, Gislon e Trupiano S.r.l.
Via Larga, 16
20122 Milano (IT)**

(54) **USE OF SPENT MEDIA OF LACTIC BACTERIA CULTURES AS ANTIMICROBIAL POST-BIOTIC AGENTS AND PHARMACEUTICAL OR NUTRACEUTICAL COMPOSITIONS CONTAINING THEM**

(57) Subject-matter of the invention is the use of spent media of lactic bacteria cultures, obtained by addition of pathogen culture derivatives as antimicrobial post-biotic agents, a process for their preparation, and the pharmaceutical and/or nutraceutical compositions comprising said antimicrobial post-biotic agents. Subject-matter of the invention is also the use of pathogen culture derivatives to obtain said antimicrobial post-biotic agents.

EP 4 599 837 A1

**Description**

**Summary of the invention**

Subject-matter of the invention is the use of spent media of lactic bacteria cultures, obtained by addition of pathogen culture derivatives as antimicrobial post-biotic agents, a process for their preparation, and pharmaceutical and/or nutraceutical compositions comprising said antimicrobial post-biotic agents. Subject-matter of the invention is also the use of pathogen culture derivatives to obtain said antimicrobial post-biotic agents.

**Technical background**

Lactic acid bacteria (LAB) are currently considered of great importance given their increasing use in improving human and animal health and nutrition. In general, lactic bacteria are found in food products (milk, yogurt, fruits, vegetables and many others), whereas some species are uniquely part of the human microbial flora, otherwise known as the "microbiota", which is of relevant importance with regards to intestinal and/or with urogenital location, eubiotic saprophytes.

In general, the use of lactic bacteria to restore natural microbial flora is by oral administration or topical application. There are multiple mechanisms by which colonization occurs, for example, competition for attachment sites or inhibition of adherence of pathogenic bacteria, or even competition for nutrients. Furthermore, by producing lactic acid, the lactic bacteria in the microbiota reduce the pH, thus creating a restrictive environment that hinders the growth of pathogenic bacteria.

It is also currently known that some, but not all, strains of lactic bacteria are able to inhibit the growth of pathogenic microorganisms when placed in the same culture. The phenomenon can be ascribed to the production of particular functional substances, (among them also the so-called bacteriocins), which are able to act on the one hand indirectly, by modulating the response of the host epithelium, and on the other hand directly, by inhibiting the growth of pathogens. The ability to produce these functional substances is inherent in the bacterium, as the genes encoding them are located on the bacterium's genome and, more rarely, on plasmids. However, in order for the strains of lactic bacteria capable of producing said particular substances to be used effectively in therapy, they must be able to maintain over time and/or increase that capability. Furthermore, it is desirable to have the ability to modulate the production of the substances above in terms of time and quantity.

EP 0 949 330, in the name of the present Applicant, describes a method for increasing the antimicrobial activity of lactic bacteria by addition of pathogen agents or pathogen culture derivatives to the growth cultures of said lactic bacteria.

**Objects of the invention**

An object of the invention is to provide the use of spent media of lactic bacteria cultures derived from the production of lactic bacteria with pathogen culture derivatives, as antimicrobial post-biotic agents.

Another object of the invention is to provide pharmaceutical or nutraceutical compositions comprising said antimicrobial post-biotic agents.

Another object of the invention is to provide the use of pathogen culture derivatives to obtain the antimicrobial agents according to the invention.

A further object of the invention is to provide a method for obtaining antimicrobial post-biotic agents, which comprises culturing lactic bacteria together with pathogen culture derivatives.

**Description of the invention**

The Applicant was able to observe that not only lactic bacteria grown in co-culture with pathogen culture derivatives exhibit relevant antimicrobial properties, but also the residual spent media from the said cultures can exert a powerful antimicrobial action.

According to one of its aspects, subject-matter of the invention is the use of spent culture media consisting of one or more strains of lactic bacteria cultured with pathogen culture derivatives, as antimicrobial post-biotic agents.

According to one of its aspects, subject-matter of the invention is a method for obtaining antimicrobial post-biotic agents comprising culturing one or more strains of lactic bacteria together with pathogen culture derivatives and isolating the spent media of said culture to provide post-biotic agents.

The expression "lactic bacteria" includes "probiotic bacteria"; preferably the lactic bacteria of the invention are probiotic bacteria.

The expression "probiotic bacteria" (or even just "probiotics") designates bacteria capable of exerting a beneficial effect on the host organism when taken in adequate amounts (Morelli L., et. Al. Gastroenterol Hepatol, 11(8):506-14, 2014)

**EP 4 599 837 A1**

Among the probiotic bacteria according to the invention, those belonging to the *Lattobacillus* and *Streptococcus* genus are preferred.

In particular, probiotic bacteria selected from *L. acidophilus, L. crispatus, L. delbrueckii, L. gasseri, L. paracasei, L. plantarum, L. reuteri, L. rhamnosus, L. salivarius, L. vaginalis* are preferred.

The preferred probiotic bacteria are *Lactobacillus salivarius* I 1794, *Lactobacillus paracasei* I *1688, Lactobacillus plantarum* P 17630, *L. paracasei* I 1687, *L. gasseri* P 17632, *L. gasseri* P 18137, *L. acidophilus* P 18806, *L. crispatus* P 17631 and *L. delbrueckii* P 18805 and mixtures thereof.

Other probiotic bacteria that may be used according to the invention are streptococci that exhibit probiotic activity, for example, *Streptococcus thermophilus*, in particular *Streptococcus thermophilus* strain P 18807.

The term "pathogens" or "pathogenic microorganisms" is meant herein to refer to microorganisms capable of inducing diseases, for example, bacteria, fungi, protozoa and the like. By way of example, pathogens include *Candida* spp., *Escherichia coli*, *Trichomonas spp*, *Streptococcus spp*, *Enterococcus spp*, *Staphilococcus spp* and *Malassezia spp*.

The expression "pathogen culture derivatives" (or similar expressions) herein means the culture residues of one or more pathogens, as defined above, where such derivatives consist of the culture supernatant of said one or more pathogens. Therefore, a pathogen culture derivative is the supernatant represented by the liquid obtained by separation of the cellular deposit of said one or more pathogens, which may be previously concentrated by centrifugation of the culture, such culture being obtained by growing one or more strains of pathogens in an appropriate culture medium and under the suitable growth conditions of said pathogens. Said supernatant is inactivated and preferably stored in any appropriate manner, for example, in a refrigerator or freezer until use. Inactivation techniques include sterilization techniques such as sterilizing filtration or other inactivation techniques that ensure that pathogen elimination is complete. Thus, pathogen culture derivatives do not contain pathogen cells, either alive or inactivated. For the term "post-biotic" or "post-biotic agent" reference is made herein to the 2019 International Scientific Association for Probiotics and Prebiotics (ISAPP) consensus document, which defines it as a preparation of inanimate microorganisms and/or their components that confers health benefit to the host.

The expression "spent media" or "spent culture media" means the liquid portion of a culture of bacteria that remains at the end of the growth of said bacteria and is depleted of the nutrients necessary for their multiplication and other essential components that were consumed during bacterial growth. Indeed, as the bacteria grow and divide in a medium, the nutrients necessary for their growth that are available in the medium are consumed, and as a result, the nutrients useful to them are depleted in that medium and the bacteria are no longer able to grow. Such media are referred to as spent media and are usually a waste material.

However, in such spent media there are metabolic products of the bacteria that can be considered functional for humans. Said media may also contain lactic bacteria cells as a residue from the centrifugation step.

Such residual lactic bacteria are to be considered a waste, as they escaped the collection during centrifugation and consist of lactic bacteria only. Therefore, the inactivation step provided for the treatment of the media of the invention is indeed to inactivate any residual bacterial cells to a value permissible as a contaminant.

During the growth process of the lactic bacteria, said bacteria produce functional substances. By "functional substances" is meant, for example, to refer to bacteriocins, metabolic enzymes, amino acids and peptides, short-chain fatty acids, vitamins, antioxidants, anti-inflammatory and immune-modulating agents, and exopolysaccharides.

As previously mentioned, EP 0 949 330, in the name of the present Applicant, describes a method for increasing the antimicrobial activity of lactic bacteria by addition of pathogen culture derivatives to the growth cultures of said lactic bacteria.

The Applicant observed that, when cultured with pathogen culture derivatives as defined herein, the lactic bacteria not only increase their antimicrobial activity as described in EP 0 949 330, but also release bioactive compounds having marked antimicrobial activity into the medium of said culture.

Thus, this observation led to considering the spent media of said lactic bacteria cultures as antimicrobial post-biotic agents instead of waste material, as was previously the case. According to the present invention, spent media derived from lactic bacteria cultures as defined above constitute post-biotic agents preferably having antimicrobial action.

Any strain or mixture of strains of lactic bacteria can be subjected to the method of the invention to provide the post-biotic agents of the invention.

Preferably, the strains of lactic bacteria to be subjected to the method of the invention are isolated from the normal intestinal and/or vaginal bacterial flora of a healthy human subject, according to techniques known to the skilled in the art. Preferably, such lactic bacteria are subjected to appropriate pretreatments according to conventional methods that allow the preservation of the strains until use, such as, but not limited to, the cryo-preservation technique. According to a preferred embodiment, the post-biotic spent media of the present invention are obtained by a method which allows inducing and/or enhancing and/or maintaining the capability of producing functional substances in lactic bacteria, through their growth in the presence of a pathogen culture derivative, as defined above. Said method allows inducing strains of lactic bacteria having a recognized microbicidal activity (i.e., capable of producing functional substances) to maintain over time and/or increase their production of functional substances, but it also allows inducing such production in those lactic bacteria that, while implicitly having such production capability, are unable to perform it.

For their use as antimicrobial post-biotic agents, the spent media of the invention, which consist of the liquid portion of the above-defined nutrient-depleted culture, are preferably inactivated according to any known technique and preferably concentrated. More preferably, the spent media are inactivated according to any known technique and then lyophilized.

Inactivation, if any, is carried out to ensure that there are no residual living lactic bacteria in the spent medium, above the limit allowed as a contaminant.

The culture media used according to the invention must be compatible with the administration to humans and/or animals.

By way of example, all media containing food-grade ingredients may be used. Thus, the raw materials constituting said media must meet high standards for what concerns hygiene, food safety and absence of harmful substances, being therefore suitable for human consumption.

In general, the spent media of the invention are preferably subjected to such inactivation processes to make them safe and able to be administered to humans and/or animals, for example, by centrifugation, filtration and/or sterilization by chemical or physical processes well known to the skilled in the art.

According to a preferred embodiment, subj ect-matter of the invention is a method for preparing an antimicrobial post-biotic agent, comprising the following steps:

(i) providing an appropriate culture medium with one or more lactic bacteria;
(ii) adding a pathogen culture derivative to the culture medium of step (i);
(iii) growing said lactic bacteria in said culture medium (ii) under appropriate conditions;
(iv) removing said lactic bacteria from said culture medium to obtain a spent medium;
(v) optionally inactivating the spent medium obtained in step (iv), optionally concentrating and/or lyophilizing it, to obtain the antimicrobial post-biotic agent according to the invention.

Steps (i) and (ii) can be reversed.

As mentioned, the culture medium of step (i) must be compatible with the administration to humans and/or animals and is selected depending on the strains of lactic bacteria to be cultured; it can be, for example, the MRS medium (de Man J. C., Rogosa, M. and M.E. Sharpe, Journal of Applied Bacteriology, 23, 130-135, 1960).

The lactic bacteria are added to the culture medium at a concentration of $10^4$ to $10^{15}$ CFU (Colony Forming Units)/ml and in an amount between 0.5 and 10% to the volume of the medium contained in the production fermenter (said percentage being expressed as a volume to the total volume of the culture medium/lactic bacteria mixture).

In step (iii) the lactic bacteria are allowed to grow as long as necessary, for example for 10 to 30 hours, preferably around 14 to 24 hours, for example 14 to 20 hours, in the presence of 0.01% to 5.00% of the culture derivative of a pathogen (said percentage being expressed in volume to the total volume of the culture medium mixture).

In step (iv) the activated lactic bacteria are removed from the culture medium according to known techniques, for example by filtration or centrifugation, and the spent medium thus obtained is preferably inactivated and, if desired or necessary, further processed, for example lyophilized, according to methods known in the art.

The spent media according to the invention have proven to be very effective as antimicrobial post-biotic agents and are useful in the treatment of diseases related to the presence of pathogen agents.

According to another of its aspects, subject-matter of the invention is an antimicrobial post-biotic agent obtained by the preparation method described above.

According to another of its aspects, subject-matter of the invention are the antimicrobial post-biotic agents, as defined herein, in therapy, more specifically for the treatment and/or prevention of diseases related to the presence of pathogen agents.

By "diseases related to the presence of pathogen agents" or "diseases caused by the presence of pathogen agents" (and similar expressions) is meant herein to refer to diseases caused by an infectious agent such as a pathogenic bacterium or fungus.

Examples of such diseases include, for example, urinary tract infections (UTIs), in which E. coli is the main responsible factor for about 85% of cases of uncomplicated cystitis. Other bacteria that can take over the native microbial flora and participate in urinary tract infection are of the Streptococcus (S. agalactie) or Staphylococcus (S. aureus) genus (5-10% of UTIs). Candidiasis, which in 85-90% of cases can be traced to the *Candida albicans* fungus (hence the name), is a physiological member of the vaginal microbiota although usually present in low concentrations. Other species of *Candida* non-albicans include *C. glabrata*, *C. krusei*, *C. parapsilosis* and *C. tropicalis.*

The antimicrobial post-biotic agents of the invention that are active against pathogens are not necessarily related to the pathogen used for the preparation of the pathogen culture derivative. In other words, a post-biotic agent of the invention, obtained through the use of a derivative of a pathogen agent "X", can be used as an antimicrobial agent also to treat diseases caused by pathogens other than said pathogen "X".

Further subject-matter of the present invention are the pharmaceutical, dietary, food or nutraceutical compositions comprising one or more antimicrobial post-biotic agents as defined herein, optionally in combination with at least one pharmaceutically or otherwise physiologically acceptable carrier and/or excipient. Said compositions may also be in the form of medicinal, dietary products and medical devices.

Compositions comprising one or more post-biotic agents as defined herein are an additional subject-matter of the invention.

Said compositions can be used by oral, buccal or topical route and are advantageously in unit dosage form or multi-dose form.

Excipients will preferably be selected to promote the stability of said compositions. For example, inert excipients that do not react with post-biotic agents and do not promote the growth of microorganisms within the composition itself.

By way of example, the compositions of the invention for oral or buccal administration may be in tablet form, chewable tablets, gummy oral forms, hard capsules, soft capsules, powders, granules, or in liquid form, such as solutions or suspensions. Such compositions may be prepared as well known in the art and may comprise conventional excipients and carriers that are pharmaceutically or otherwise conventional physiologically acceptable.

By way of illustration, the solid forms may include starches, cellulose and derivatives thereof; lubricants such as talc, stearic acid, polyethylene glycol or magnesium stearate; diluents such as talc, powdered cellulose, lactose, starches such as corn or wheat starch, mannitol, sorbitol; disaggregating agents such as microcrystalline cellulose or crospovidone; ligands such as methylcellulose, sodium carboxymethylcellulose, alginic acid, alginates; sweeteners such as sucrose, dextrose, mannitol, saccharin, xylitol, sorbitol; or flavorings such as synthetic or natural oils.

Buccal compositions are particularly suitable for the treatment of oral mucosal diseases, such as, for example, mouth ulcers, stomatitides, and the like.

For topical administration, the compositions of the invention may comprise excipients and carriers suitable for topical application. Such compositions may be, for example, in the form of cream (oil in water or water in oil), emulsion, gel, solution, oil, powder, ointments, pastes, foams and sprays. Scalp-cleansing shampoos can also be prepared, as well as foams for quick and easy applications. Alternatively, said compositions can be formulated in combination with a patch and thus be in the form of a patch to be applied to the skin.

The compositions of the invention can be packaged in single-dose (dosage unit) or multi-dose containers.

The compositions of the invention may contain 50 mg to 2 g, preferably 100 mg to 1 g, of antimicrobial post-biotic agent according to the route of administration for which the invention is intended.

Alternatively, the antimicrobial post-biotic agents for use according to the invention can be used as is, for example and preferably, in lyophilized form by supplementing them to foods and beverages, such as, for example, yogurt, milk, fruit or vegetable juices or extracts, creams, jellies and the like.

According to another of its aspects, subject-matter of the invention is a method for the treatment and/or prevention of diseases related to the presence of pathogen agents, which comprises administering, to a subject in the need thereof, an effective amount of an antimicrobial post-biotic agent as defined herein or a composition comprising it.

The subject to be treated according to the invention is a mammal, in particular but not only, human beings.

The experimental section below describes the invention in more detail for illustrative and nonlimiting purpose only.

**Experimental section**

Example 1

Preparation of a *Candida* supernatant

A sample of pathogen (*Candida*) cultured overnight was diluted to $1 \times 10^7$ CFU/ml and incubated at 37°C for another 24 hours in anaerobiosis. Next, the *Candida* culture supernatant (CCS) has been collected by centrifugation at 5000 rpm for 10 min and then filtered and sterilized with a 0.22 $\mu$m pore size filter.

Example 2

Preparation of an antimicrobial post-biotic agent according to the invention

100-200 $\mu$l of a freezing vial containing a selected strain of lactic bacteria, preserved in this way, were cultured at 37°C overnight in 10 ml of MRS broth. 100 $\mu$l of the above suspension was placed in 9.9 ml of MRS broth and incubated for 24 h at 37°C. The same strain of lactic bacteria was previously treated, according to the procedure of the invention, by culturing it in the presence of *Candida* supernatant from Example 1, in concentrations ranging from 0.01% to 5.00% (w/v)).

The medium used for the culture, i.e. MRS, has the following composition:

| Meat extract | 8 g |
|---|---|
| Yeast extract | 4 g |
| Proteose peptone | 10 g |
| Sodium acetate | 5 g |
| Dibasic ammonium acetate | 2 g |
| Dibasic potassium phosphate | 2 g |
| Glucose | 20 g |
| Saline solution A | 5 ml |
| Tween 80 | 1 ml |
| Double-distilled water | 1000 ml |

At the end of culture, the cell mass is separated from the culture medium by centrifugation and said medium is subjected to inactivation.

Example 3

Example of composition of a dosage unit in capsule form

| Composition | | |
|---|---|---|
| *Post-biotic from Lactobacillus crispatus* P 17631 grown in co-culture with supernatant of pathogen such as *Candida* | mg 153 | Active ingredient |
| Excipients | | |
| Mannitol | mg 57 | Diluent |
| Corn starch | mg 77 | Diluent |
| Sodium croscarmellose | mg 6 | Disintegrant |
| Magnesium stearate | mg 4 | Lubricant |
| Colloidal hydrated silica (levilite) | mg 3 | Drying |

Example 4

Example of composition of a dosage unit in tablet form

| Composition | | |
|---|---|---|
| *Post-biotic from Lactobacillus crispatus* P 17631 grown in co-culture with supernatant of pathogen such as *Candida* | mg 173 | Active ingredient |
| Excipients | | |
| Pre-gelled starch | mg 85 | Diluent |
| Maltodextrins | mg 115 | Thickener |
| Inulin | mg 115 | Stabilizer |
| Magnesium stearate | mg 4 | Lubricant |
| Compritol | mg 8 | Compressibility |

Example 5

Experimental tests

Testing of antimicrobial activity

*Lactobacillus crispatus* P 17631, set to grow with the inactivated supernatant of *Candida albicans*, was used to produce the post-biotic P 17631. The strain was maintained in MRS medium [Merck, Darmstadt, Germany] at - 20°C supplemented with 20% [v/v] glycerol [Merck, Darmstadt, Germany] and then resuscitated in MRS medium (Merck, Darmstadt, Germany) according to the method previously described by Lim, Y.S. et al. 2003. The post-biotic produced by *L. crispatus* P 17631 was referred to as post-biotic P 17631, in this study. The preparation of post-biotic P 17631 was performed according to the method of Ooi, M.F. et al. 2021. The post-biotic P 17631 was collected by centrifugation (10,000×g for 15 min at 4 °C) and stored at 4 °C for determining the antimicrobial activity. The antimicrobial activity of post-biotic P 17631 was determined by using an Agar well diffusion assay modified without neutralizing the pH of post-biotic P 17631. To dilute the post-biotic P 17631, a serial double dilution ($2^0$ to $2^{-5}$) was performed by using a sterile 0.85% (w/v) NaCl solution. The diluted post-biotic P 17631 (20 μL) was then allowed to diffuse around a well (5.0 mm in diameter) pre-drilled in MRS agar medium, followed by overlaying with 3 mL of soft agar inoculated with 1% (v/v) *C. albicans* (OD600nm was adjusted to 1.0) before incubating at 30 °C for 24 h. A clear zone of inhibition with a diameter greater than 1 cm (including the 0.5 cm diameter of the well) was considered positive antimicrobial activity. The antimicrobial activity was expressed as a modified arbitrary unit (MAU/mL) by using the following equation:

**Modified Arbitrary Units (MAU/mL)**

$$\frac{\text{Result of the maximum dilution in the inhibition zone (AU)}}{\text{Volume of post-biotic (mL)}} \text{ x Diameter of the inhibition zone}$$

Determination of lactate concentration and pH

The lactate concentration of post-biotic P 17631 obtained as described above (Ooi, M.F. et al. 2021) was determined with the Pico Trace Glucose Analyser (Trace Analytics GmbH, Germany) according to the manufacturer's instructions, and the pH of post-biotic P 17631 was determined with a pH-meter (Mettler Toledo, USA).

Results

The post-biotic P 17631 showed a powerful antimicrobial activity in the above test, confirming that the spent media derived from lactic bacteria cultures supplemented with pathogen culture derivatives can be successfully used as antimicrobial post-biotic agents.

Example 6

The post-biotic of Example 5 was further tested according to the analytical methodology of determining the minimum inhibitory concentration (MIC) defined as the lowest concentration of an antimicrobial agent that inhibits the visible growth of a microorganism.
The *Candida albicans* pathogen was inoculated into test tubes containing culture broth suitable for pathogen development.
Next, these tubes were supplemented with increasing amounts of post-biotic, in the order of doubling for each concentration jump; we then proceeded to the incubation phase under temperature and aerobic/anaerobic conditions as required by the pathogen tested.
At the end of the incubation period, it was possible to display the last tube that showed visible growth of the pathogen.
The first tube contained only the pathogen as a control.
Subsequent tubes contained the same concentration in colony-forming units of the pathogen and also an increasing amount, tube by tube, of the post-biotic.
The same test was performed in parallel by adding the lactobacillus culture supernatant from Example 5, grown in the absence of pathogen derivative to have a control.
At the end of the test, it could be observed that the post-biotic of Example 5 showed a significantly higher degree of efficacy than the control.

**EP 4 599 837 A1**

**Bibliography**

1) Hill C, Guarner F, Reid G, Gibson GR, Merenstein DJ, Pot B, Morelli L, Canani RB, Flint HJ, Salminen S, Calder PC, Sanders ME. Expert consensus document. The International Scientific Association for Probiotics and Prebiotics consensus statement on the scope and appropriate use of the term probiotic. Nat Rev Gastroenterol Hepatol. 2014 Aug;11(8):506-14. doi: 10.1038/nrgastro.2014.66. Epub 2014 Jun 10. PMID: 24912386.

2) Ooi MF, Foo HL, Loh TC, Mohamad R, Rahim RA, Ariff A. A refined medium to enhance the antimicrobial activity of postbiotic produced by Lactiplantibacillus plantarum RS5. Sci Rep. 2021 Apr 7;11(1):7617. doi: 10.1038/s41598-021-87081-6. PMID: 33828119; PMCID: PMC8027010.

3) Lim, Y.S. Isolation of bacteriocinogenic lactic acid bacteria and purification of selected bacteriocins from traditional fermented foods. Master Thesis, Universiti Putra Malaysia, Mlaysia. (2003).

**Claims**

1. Spent media of lactic bacteria cultures supplemented with pathogen culture derivatives, for use as antimicrobial post-biotic agents, in the treatment and/or prevention of diseases caused by the presence of pathogen agents.

2. Spent media for use according to claim 1, **characterized in that** said pathogens include *Candida* sp., *Escherichia coli, Trichomonas vaginalis,* $\beta$-hemolytic *Streptococcus, Enterococcus sp* and *Malassezia furfur.*

3. A method for preparing an antimicrobial post-biotic agent, comprising culturing one or more strains of lactic bacteria together with derivatives of pathogen cultures and isolating the spent media of said culture as antimicrobial post-biotic agent.

4. The method according to claim 3, comprising the following steps:

   (i) providing an appropriate culture medium with one or more lactic bacteria;
   (ii) adding a pathogen culture derivative to the culture medium of step (i);
   (iii) growing said lactic bacteria in said culture medium, under appropriate conditions;
   (iv) removing said lactic bacteria from said culture medium to obtain a spent medium;
   (v) optionally inactivating the spent medium obtained in step (iv) and optionally concentrating or lyophilizing it, to obtain the antimicrobial post-biotic agent according to the invention.

5. An antimicrobial post-biotic agent obtained according to the preparation method of claim 3 or 4.

6. A pharmaceutical, dietary, food or nutraceutical composition comprising one or more antimicrobial post-biotic agents according to claim 5, optionally in combination with at least one pharmaceutically or otherwise physiologically acceptable carrier and/or excipient.

7. The composition according to claim 6, **characterized in that** it is an oral, buccal or topical composition.

8. The composition according to claim 6 or 7, for use in therapy.

9. The composition according to claim 6 or 7, for use in the treatment and/or prevention of diseases caused by the presence of pathogen agents.

10. The composition for use according to claim 9, **characterized in that** said pathogens include *Candida* sp., *Escherichia coli, Trichomonas vaginalis,* $\beta$-hemolytic Streptococcus, *Enterococcus sp* and *Malassezia furfur.*

Europäisches
Patentamt

European
Patent Office

Office européen
des brevets

# EUROPEAN SEARCH REPORT

**Application Number**

EP 25 15 7215

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 0 949 330 A2 (PROGE FARM SRL [IT]) 13 October 1999 (1999-10-13) | 3-5 | INV. A61K35/74 |
| Y | * the whole document * | 1-10 | A23L33/135 A61K35/744 |
| Y | US 2022/347257 A1 (CELLA MONICA ANGELA [CA]) 3 November 2022 (2022-11-03) * claims 1-33 * | 1-10 | A61K35/747 C12N1/20 |
| Y | US 2024/024378 A1 (SIMMONS SHERI [US] ET AL) 25 January 2024 (2024-01-25) * paragraph bridging cols. 13 and 14 * | 1-10 | |

**TECHNICAL FIELDS SEARCHED (IPC)**

A61K
A61P
C12R
A23L
C12N

The present search report has been drawn up for all claims

| Place of search | Date of completion of the search | Examiner |
|---|---|---|
| Munich | 18 June 2025 | Schnack, Anne |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 15 7215

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2025

| Patent document cited in search report | | Publication date | Patent family member(s) | | Publication date |
|---|---|---|---|---|---|
| EP 0949330 | A2 | 13-10-1999 | EP | 0949330 A2 | 13-10-1999 |
| | | | EP | 1894996 A1 | 05-03-2008 |
| | | | IT | MI980775 A1 | 10-10-1999 |
| | | | US | 6342366 B1 | 29-01-2002 |
| US 2022347257 | A1 | 03-11-2022 | AU | 2020299061 A1 | 10-02-2022 |
| | | | BR | 112022000041 A2 | 15-03-2022 |
| | | | CA | 3145739 A1 | 07-01-2021 |
| | | | CN | 114786703 A | 22-07-2022 |
| | | | EP | 3993819 A1 | 11-05-2022 |
| | | | JP | 2022540096 A | 14-09-2022 |
| | | | US | 2022347257 A1 | 03-11-2022 |
| | | | WO | 2021000046 A1 | 07-01-2021 |
| US 2024024378 | A1 | 25-01-2024 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0949330 A

**Non-patent literature cited in the description**

- **MORELLI L.** *Gastroenterol Hepatol*, 2014, vol. 11 (8), 506-14
- **DE MAN J. C.** ; **ROGOSA, M.** ; **M.E. SHARPE**. *Journal of Applied Bacteriology*, 1960, vol. 23, 130-135
- **HILL C** ; **GUARNER F** ; **REID G** ; **GIBSON GR** ; **MERENSTEIN DJ** ; **POT B** ; **MORELLI L** ; **CANANI RB** ; **FLINT HJ** ; **SALMINEN S**. Expert consensus document. The International Scientific Association for Probiotics and Prebiotics consensus statement on the scope and appropriate use of the term probiotic.. *Nat Rev Gastroenterol Hepatol.*, 10 June 2014, vol. 11 (8), 506-14
- **OOI MF** ; **FOO HL** ; **LOH TC** ; **MOHAMAD R** ; **RAHIM RA** ; **ARIFF A**. A refined medium to enhance the antimicrobial activity of postbiotic produced by Lactiplantibacillus plantarum RS5. *Sci Rep.*, 07 April 2021, vol. 11 (1), 7617
- Isolation of bacteriocinogenic lactic acid bacteria and purification of selected bacteriocins from traditional fermented foods. **LIM, Y.S.** Master Thesis. Universiti Putra Malaysia, 2003